# EUROPEAN PATENT APPLICATION

(11) **EP 2 767 222 A1**
(43) Date of publication of application: **20.08.2014**
(21) Application number: 14151236.8
(22) Date of filing: 15.01.2014
(51) Int. Cl.: A61B 5/00, A61B 5/0464

(54) **Implantable heart monitoring device**

(30) Priority: 19.02.2013 US 201361766139 P
(71) Applicant: BIOTRONIK SE & Co. KG, 12359 Berlin (DE)
(72) Inventor: Garner, Garth, Tigard, OR Oregon 97224 (US); Moulder, J. Christopher, Portland, OR Oregon 97202 (US); Kraetschmer, Hannes, West Linn, OR Oregon 97068 (US); Whittington, R. Hollis, Portland, OR Oregon 97202 (US); Müssig, Dirk, West Linn, OR Oregon 97068 (US); Vennelaganti, Swetha, Lake Oswego, OR Oregon 97035 (US)
(74) Representative: Lamprecht, Frank

(57) **Abstract**

The invention regards an implantable heart monitoring device (10) comprising a sensing unit (58, 66), a signal quality analysis unit (96), and an evaluation unit (98). The sensing unit (58, 66) can be connected or is connected to at least one electrode (22, 24, 30, 32), which is configured for picking up electric potentials. The sensing unit (58, 66) is adapted to process electrical signals corresponding to said electric potentials, to detect signal components representing a myocardial contraction, and to generate sense signals comprising events (102, 104) corresponding to myocardial contractions. The signal quality analysis unit (96) is configured to determine whether a noise condition (NC) and/or a low signal indication is present for an event (102, 104) and to generate a noise condition and/or low signal indication signal. The evaluation unit (98) is connected or connectable to said sensing unit (58, 66) and said signal quality analysis unit (96) and configured to evaluate sense signals and noise condition and/or low signal indication signals. The evaluation unit (98) is further configured to treat a detected event (104) as non-valid if a noise condition (NC) and/or low signal indication signal is present for that event (104) and to use only intervals (106) defined by two consecutive events (102) which do not have a noise condition (NC) and/or a low signal indication to determine a rate of events of the sense signal.

## Description

The present invention relates to an implantable heart monitoring device.

A implantable heart monitoring device may be a standalone device or part of an implantable heart stimulator such as dual-chamber (RA-RV), three-chamber (BiA-RV, or RA-BiV), or four-chamber (BiA-BiV) implantable cardiac devices including pacemakers, defibrillators and cardiovertors, which monitor and, if needed, stimulate cardiac tissue electrically to control the patient's heart rhythm.

Implantable heart stimulators can be used for cardiac rhythm management (CRM) for treating a variety of heart functional and rhythm disorders including but not limited to bradycardia, tachycardia or fibrillation by way of electric stimulation pulses delivered to the heart tissue, the myocardium. A sufficiently strong stimulation pulse outside a heart chamber's refractory period leads to excitation of the myocardium of that heart chamber, which in turn is followed by a contraction of the respective heart chamber.

Tachycardia is a rhythm disorder that comprises a high cardiac rate while bradycardia refers to too low a heart rate. Fibrillation is a state wherein the heart myocytes are depolarizing in a non-coordinated manner, preventing the heart from pumping blood.

Depending on the disorder to be treated, such heart stimulator generates electrical stimulation pulses that are delivered to the heart tissue (myocardium) of a respective heart chamber according to an adequate timing regime. Delivery of stimulation pulses to the myocardium is usually achieved by means of an electrode lead that is electrically connected to a stimulation pulse generator inside a heart stimulator's housing and that carries a stimulation electrode in the region of its distal end. A stimulation pulse also is called a pace. Similarly, pacing a heart chamber means stimulating a heart chamber by delivery of a stimulation pulse.

In order to be able to sense the contraction of a heart chamber, which occurs naturally without artificial stimulation and which is called an intrinsic contraction, the heart stimulator usually includes at least one sensing stage that is connected to a sensing electrode and said electrode is placed in or near the heart chamber. An intrinsic excitation of a heart chamber results in characteristic electrical potentials that can be picked up via the sensing electrode and that can be evaluated by the sensing stage in order to determine whether an intrinsic excitation, or intrinsic event, has occurred.

Usually, a heart stimulator features separate stimulation pulse generators for each heart chamber to be stimulated. Therefore, in a dual chamber pacemaker, usually an atrial and a ventricular stimulation pulse generator for generating atrial and ventricular stimulation pulses are provided. Delivery of an atrial or a ventricular stimulation pulse causing an artificial excitation of the atrium or the ventricle, respectively, is called an atrial stimulation event AP (atrial paced event) or a ventricular stimulation event VP (ventricular paced event), respectively.

Similarly, common heart stimulators feature separate sensing channels for each heart chamber to be of interest. In a dual chamber pacemaker usually two separate sensing channels, an atrial sensing channel and a ventricular sensing channel, are provided that are capable of detecting intrinsic atrial events AS (atrial sensed event) or intrinsic ventricular events VS (ventricular sensed event), respectively.

In a heart cycle, an excitation of the myocardium leads to a depolarization of the myocardium that leads to a contraction of the heart chamber. If the myocardium is fully depolarized it is not susceptible to further excitation and is thus refractory. Thereafter, the myocardium repolarizes and thus relaxes and the heart chamber expands again. In a typical intracardiac electrogram (IEGM) depolarization of the ventricle corresponds to a signal known as the "R-wave". The "R-wave" is commonly preceded by a downward deflection, known as the "Q-wave" and followed by another downward deflection, known as the "S-wave". Normal "Q-waves" represent depolarization of the interventricular septum. Any combination of Q-wave, R-wave, or S-wave is called QRS complex. The repolarization of the ventricular myocardium coincides with a signal known as the "T-wave". Atrial depolarization is manifested by a signal known as the "P-wave".

In a healthy heart, initiation of the cardiac cycle normally begins with depolarization of the sinoatrial (SA) node. This specialized structure is located in the upper portion of the right atrium wall and acts as a natural "pacemaker" of the heart. In a normal cardiac cycle and in response to the initiating SA depolarization, the right atrium contracts and forces the blood that has accumulated therein into the ventricle. The natural stimulus causing the right atrium to contract is conducted to right ventricle via the atrioventricular node (AV node) with a short, natural delay, the atrioventricular delay (AV-delay). Thus, a short time after the right atrial contraction (a time sufficient to allow the bulk of the blood in the right atrium to flow through the one-way valve into the right ventricle), the right ventricle contracts, forcing the blood out of the right ventricle to the pulmonary artery. A typical time interval between contraction of the right atrium and contraction of the right ventricle might be 100 ms; a typical time interval between contraction of the right ventricle and the next contraction of the right atrium might be 800 ms. Thus, it is a right atrial contraction (A), followed a relatively short time thereafter by a right ventricle contraction (V), followed a relatively long time thereafter by the next right atrial contraction, that produces the desired AV synchrony. Where AV synchrony exists, the heart functions very efficiently as a pump in delivering life-sustaining blood to body tissue; where AV synchrony is absent, the heart functions as an inefficient pump, largely because the right ventricle is contracting when it is not filled with blood.

Similarly, the left ventricle contracts in synchrony with right atrium and the right ventricle with a positive or negative time delay between a right ventricular contraction and a left ventricular contraction.

A pacemaker generally induce a contraction of a heart chamber by delivery of a stimulation pulse (pacing pulse) to said chamber when no natural (intrinsic) contraction of said chamber occurs in due time. A contraction of a heart chamber often is called "event." Because a contraction may be an intrinsic contraction, which can be sensed by a sensing stage of a pacemaker, such an event is called a sensed event. A contraction due to delivery of a stimulation pulse is called a paced event. A sensed event in the atrium is called AS, a paced atrial event is called AP. Similarly, a sensed event in the ventricle is called VS and a paced ventricular event is called VP.

To mimic the natural behavior of a heart, a dual-chamber pacemaker provides for an AV-delay timer to provide for an adequate time delay (atrioventricular delay, AV-delay, AVD) between a natural (intrinsic) or a stimulated (paced) right atrial contraction and a right ventricular contraction. In a similar way a biventricular pacemaker provides for an adequate time delay (VV-delay, VVD) between a right ventricular contraction and a left ventricular contraction.

The time delay for a left ventricular (stimulated, paced) contraction may be timed from a scheduled right ventricular contraction which has not yet occurred or from a natural (intrinsic) or a stimulated (paced) right atrial contraction. In the latter case a left ventricular stimulation pulse is scheduled by a time interval AVD + VVD.

To deal with possibly occurring natural (intrinsic) atrial or ventricular contractions, a demand pacemaker schedules a stimulation pulse for delivery at the end of the AV-delay or the VV-delay, respectively. The delivery of said stimulation pulse is inhibited, if a natural contraction of the heart chamber to be stimulated is sensed within the respective time delay.

A natural contraction of a heart chamber can be similarly detected by evaluating the electrical signals sensed by the sensing channels. In the sensed electrical signal the depolarization of an atrium muscle tissue is manifested by occurrence of a P-wave. Similarly, the depolarization of ventricular muscle tissue is manifested by the occurrence of an R-wave. The detection of a P-wave or an R-wave signifies the occurrence of intrinsic atrial, AS, or ventricular, VS events, respectively.

A dual chamber pacemaker featuring an atrial and a ventricular sensing stage and an atrial and a ventricular stimulation pulse generator can be operated in a number of stimulation modes like VVI, wherein atrial sense events are ignored and no atrial stimulation pulses are generated, but only ventricular stimulation pulses are delivered in a demand mode, AAI, wherein ventricular sense events are ignored and no ventricular stimulation pulses are generated, but only atrial stimulation pulses are delivered in a demand mode, or DDD, wherein both, atrial and ventricular stimulation pulses are delivered in a demand mode. In such DDD mode of pacing, ventricular stimulation pulses can be generated in synchrony with sensed intrinsic atrial events and thus in synchrony with an intrinsic atrial rate, wherein a ventricular stimulation pulse is scheduled to follow an intrinsic atrial contraction after an appropriate atrioventricular delay (AV-delay; AVD), thereby maintaining the hemodynamic benefit of atrioventricular synchrony.

To allow for correct diagnosis with a implantable cardiac monitoring device and for an effective stimulation with an implantable heart stimulator the sensing stage of the implantable cardiac monitoring device or the implantable heart stimulator has to identify if a heart functional or rhythm disorder is present in the heart beat.

WO 2012/015498 Al presents a medical system and method to reject undersensing in a signal indicative of cardiac activity, e.g., ECG. The medical system detects at least one of a asystole or a bradycardia based on the comparison of the amplitude of the signal to a first threshold. The medical system can determine whether the detection of the asystole or the bradycardia is false based on the comparison of an amplitude of a detected R-wave in the signal to at least a second threshold.

In EP 1 237 622 B1 an external defibrillator with an electrode, a sense circuit, a cardiac arrhythmia detector, a microprocessor-based controller, and a therapy delivery circuit is presented. The electrode is coupled externally to a body. The sense circuit is coupled to the electrode to sense a physiological signal indicative of intrinsic cardiac activity. The cardiac arrhythmia detector is coupled to the sense circuit to detect a cardiac arrhythmia based on the physiological signal. The cardiac detector includes a rate detector, which detects a first average intrinsic cardiac rate for a predetermined number N of cardiac events and a second average by dropping measurements related to one of the events, with the second average being taken for the remaining N-1 cardiac events. The therapy delivery circuit delivers electrical therapy pulses to a patient to correct abnormal cardiac arrhythmia.

It is an object of the invention to provide an improved apparatus and a method for monitoring electric potentials corresponding to myocardial contraction, i.e., a cardial beat or heart beat.

In one embodiment an implantable heart monitoring device comprising a sensing unit, a signal quality analysis unit, and an evaluation unit. The sensing unit can be connected or is connected to at least one electrode. The at least one electrode is configured to pick up electric potentials. The sensing unit is adapted to process electrical signals corresponding to said electric potentials, to detect signal components representing a myocardial contraction, and to generate sense signals representing detected events corresponding to myocardial contractions. The signal quality analysis unit is configured to determine whether a noise condition (NC), a low signal indication or both are present for a detected event and to generate a noise condition and/or low signal indication signal. The evaluation unit is connected or connectable to said sensing unit and said signal quality analysis unit and configured to evaluate sense signals and noise condition and/or low signal indication signals. The evaluation unit is further configured to treat a detected event as non-valid or invalid, respectively, if a noise condition (NC) and/or low signal indication signal is present for that event and to use only intervals defined by two consecutive events which do not have a noise condition (NC) and/or a low signal indication to determine a rate of events of the sense signal.

Low signal quality due to noise or due to a low signal strength may affect the reliability of the sensing of events, in particular with respect to the moment of occurrence of the event. An invalid event is an event that has an associated noise condition (NC) or low signal indication. An interval defined by an invalid event has a large error, as the moment of occurrence of the invalid event cannot be defined with high certainty, leading to large errors for interval durations and therefore these intervals are unusable for the determination of a rate of events, i.e., unusable or unused intervals.

It is to be noted that rhythm classification can equally be based on an analysis of interval duration or an analysis of rate of events, because an interval is the inverse of a rate of events.

The signal quality analysis unit can be integral part of the sensing unit thus forming a combined sensing/signal quality analysis unit that detects signal components that represent cardiac events and generates a sense signal, e.g., a marker signal and that flags a sense signal as noisy signal or low signal if a noise condition or a low signal indication is detected, respectively. The combined sensing and signal quality analysis unit may also be configured to generate a flag only for the events with a noise condition and/or a low signal indication with respect to an individual sense signal. Alternatively, the signal quality analysis unit may be implemented as a separate unit or it can be part of the evaluation unit.

The evaluation unit treats those sense signals or events for which a noise condition or a low signal indication was detected as "invalid" sense signals or "invalid" events. The evaluation unit classifies those events of sense signals for which a noise condition or a low signal condition was detected as "invalid" events. In this way high ventricular rate, bradycardia, and asystole can be detected with a high specificity.

In a preferred embodiment the sensing unit of the implantable heart monitoring device is a ventricular sensing unit configured to detect ventricular events.

The evaluation unit is preferably configured to execute various monitoring and stimulation algorithms. The algorithms can be executed in parallel or serially. The evaluation unit can be configured to use parameters, e.g., rate of events determined from used intervals of the sense signal, interval duration, or other parameters, determined from a combined sense signal from all electrodes for all algorithms executed in parallel. Alternatively the evaluation unit can also determine individual parameters, e.g., from sense signals from different electrodes, to be used for different algorithms. As an example an evaluation unit can run a monitoring algorithm for each of two ventricles of the heart, with parameters determined from a left ventricle for the monitoring algorithm of the left ventricle and parameters determined from a right ventricle for the monitoring algorithm of the right ventricle.

Preferably the evaluation unit of the implantable heart monitoring device is configured to detect a high ventricular rate, if a high rate detection counter exceeds a predetermined threshold. The evaluation unit is preferably configured to increment the high rate detection counter each time the determined rate of events in a used interval exceeds a predetermined threshold of a high ventricular rate detection limit. The used intervals are defined by two consecutive events which do not have a noise condition (NC) and/or a low signal indication. The predetermined threshold of the high ventricular rate detection limit may be in the range from 150 to 200 bpm and is preferably adjustable in steps of 10 bpm. A default value for the predetermined threshold of the high ventricular rate detection limit may for example be 180 bpm. The predetermined threshold of the high rate detection counter can be in the range from 4 to 16 and is preferably adjustable in steps of 4. A default value for the predetermined threshold of the high rate detection counter can for example be 8. The evaluation unit is preferably configured to decrement the high rate detection counter each time the determined rate of events in a used interval is below the predetermined threshold of the high ventricular rate detection limit. The evaluation unit can also be configured to decrement the high rate detection counter each time an event has a noise condition (NC) and/or a low signal indication associated with it, meaning an invalid event. In an alternative embodiment the high rate detection counter remains unchanged for invalid events. The evaluation unit can also be configured to decrement the high rate detection counter for each time of occurrence of an unused interval, which are intervals that comprise at least one invalid event, or to remain unchanged for unused intervals. The evaluation unit preferably executes a high ventricular rate detection algorithm to detect whether a high ventricular rate is present in the sense signals.

In a preferred embodiment the evaluation unit is configured to detect a termination of the high ventricular rate if the high ventricular rate was detected and a termination counter exceeds a predetermined threshold. Alternatively the evaluation unit can also be configured to detect the termination of the high ventricular rate if the termination counter is equal to or greater than the predetermined threshold value of the termination counter. The predetermined threshold of the termination counter may be in the range from 4 to 16 and is preferably adjustable in steps of 1. A default value for the predetermined threshold of the termination counter can for example be 5. Preferably the evaluation unit is configured to increment the termination counter each time the determined rate of events in a used interval is below a predetermined threshold of the high ventricular rate detection limit. The predetermined threshold of the high ventricular rate detection limit can be different compared to the detection of a high ventricular rate. In one embodiment the termination counter is set to zero each time the determined rate of events in a used interval exceeds the predetermined threshold of the high ventricular rate detection limit. Alternatively the termination counter can be decremented, e.g., by 1, 2, or another number, as long as the termination counter is above 0, each time the determined rate of events in a used interval exceeds the predetermined threshold of the high ventricular rate detection limit. The evaluation unit preferably executes a high ventricular rate termination detection algorithm to detect whether a high ventricular rate is terminated in the sense signals.

In a preferred embodiment the evaluation unit is configured to detect an asystole if a used interval is longer than a predetermined asystole interval limit. The predetermined asystole interval limit may be in the range from 2 seconds to 10 seconds and is preferably adjustable in steps of 1 second. A default value of the predetermined asystole interval limit can for example be 3 seconds. The evaluation unit preferably executes an asystole detection algorithm to detect whether an asystole is present in the sense signals.

The evaluation unit may also be configured to detect bradycardia, if an average rate of events is less than a predetermined Brady rate limit for a predetermined bradycardia duration. The predetermined Brady rate limit can be in the range from 30 to 80 bpm and is preferably adjustable in steps of 5 bpm. A default value of the Brady rate limit can for example be 40 bpm. The predetermined bradycardia duration can be in the range from 5 to 30 seconds and is preferably adjustable in steps of 5 seconds. A default value of the bradycardia duration can be for example 10 seconds. The evaluation unit preferably executes a Brady rate limit detection algorithm to detect whether bradycardia is present in the sense signals.

In a preferred embodiment the evaluation unit is configured to find a minimum number of used intervals whose sum exceeds the predetermined bradycardia duration. The sum of the used intervals may be equal to or greater than the predetermined bradycardia duration. The evaluation unit is preferably configured to determine an average duration of the used intervals in the bradycardia duration. Preferably the evaluation unit converts the average duration into an average rate of events and determines whether the average rate of events is below the predetermined Brady rate limit. If the average rate of events of the sum of used intervals exceeding the predetermined bradycardia duration is below the predetermined Brady rate limit, bradycardia is detected. In one embodiment the evaluation unit is configured to add the newest used interval to the minimum number of used intervals when the evaluation unit is in use and sense signals comprising events are received. The oldest used interval is removed from the minimum number of used intervals if the sum of the used intervals with the newest interval and without the oldest interval exceeds the predetermined bradycardia duration. In another embodiment the evaluation unit is configured to remove the oldest used interval from the minimum number of used intervals if an event has a noise condition (NC) and/or a low signal indication, i.e., if an invalid event is present in the sense signals.

In a preferred embodiment the evaluation unit is configured to detect bradycardia, if an average rate of events decreases by a predetermined percentage threshold, i.e., detection of a Brady rate drop. A change in the average rate of events can be determined by comparing a pre-interval average comprising a predetermined pre-interval number of used intervals and a post-interval average comprising a predetermined post-interval number of used intervals. The predetermined pre-interval number can for example be 32, 48, or 64. A default value for the predetermined pre-interval number is preferably 48. The predetermined post-interval number can for example be 4, 8, or 16. A default value for the predetermined post-interval number is preferably 8. The evaluation unit preferably executes a brady rate drop detection algorithm to detect whether bradycardia is present in the sense signals.

In one embodiment the evaluation unit is configured to set a number of useable intervals to the predetermined post-interval number, when bradycardia is detected. Following an invalid event the oldest used interval can be removed from the number of used intervals and the number of useable intervals can be decremented by 1. In this case a newest interval is not added to the sum of used intervals, as the newest interval is unuseable.

The evaluation unit is preferably configured to detect bradycardia, if Brady rate drop or Brady rate limit is detected. In a preferred embodiment both bradycardia detection algorithms, i.e., Brady drop rate detection algorithm and Brady rate limit detection algorithm, preferably run in parallel on the evaluation unit. Once bradycardia is detected both bradycardia detection algorithms, i.e., Brady drop rate detection algorithm and Brady rate limit, switch to a bradycardia termination detection mode. Another embodiment of the Brady rate drop detection method comprises the step of removing used intervals from the sum of used interval following a predetermined number of invalid events. The number of useable intervals can be set to 0 if an event has a noise condition (NC) and/or a low signal indication. The predetermined invalid event count may, for example, be 1, 2, 3 or 4. A default value for the predetermined invalid event count is preferably 1.

Preferably the evaluation unit is configured to detect a termination of bradycardia, if bradycardia was detected and a predetermined bradycardia termination counter exceeds a predetermined threshold. The bradycardia termination counter is preferably set to zero when bradycardia is detected. Preferably the bradycardia termination counter is incremented for each used interval shorter than the interval equivalent of the predetermined Brady rate limit. The predetermined threshold of the Brady rate limit used for Brady rate limit detection is preferably the same as the predetermined threshold used for termination of Brady rate limit and Brady rate drop. The bradycardia termination counter can be decremented if the bradycardia termination counter is greater than zero and an invalid event is detected. The bradycardia termination counter is preferably decremented by 1 for each event which has a noise condition (NC) and/or a low signal indication. The bradycardia termination counter can also be decremented by 2, 3 or another number, or the bradycardia termination counter can be set to zero. Preferably the bradycardia termination counter is set to zero for each used interval that is longer than or equal to the interval equivalent of the predetermined Brady rate limit.

Embodiments provide methods for detecting bradycardia, asystole, and/or high ventricular rate (HVR). The methods are preferably integrated or can be integrated in monitoring algorithms, stimulating algorithms and/or monitoring and stimulating algorithms, which can be executed in parallel on the evaluation unit.

A preferred embodiment of a high ventricular rate detection method comprises the following steps:
- Detect an event corresponding to a myocardial contraction, i.e. a heart beart.
- Determine whether the event is a useable event or an invalid event. The invalid event is an event that comprises a noise condition and/or a low signal indication.
- Determine a used interval if two consecutive useable events are present in the present interval.
- Increment the high rate detection counter if the used interval exceeds a predetermined duration. The used intervals are those intervals, which are defined by two consecutive events which do not have a noise condition (NC) and/or a low signal indication.
- Decrement the high rate detection counter if the used interval is below the predetermined duration.
   - Optionally the method can comprise the step of decrementing the high rate detection counter if an event has a noise condition (NC) and/or a low signal indication, meaning if the event is an invalid event.
- Detect a high ventricular rate, if the high rate detection counter exceeds a predetermined threshold.

A preferred embodiment of a high ventricular rate termination detection method comprises the following steps:
- Initialize the high ventricular rate termination detection method only if a high ventricular rate was detected and set a termination counter to zero.
- Detect an event corresponding to a myocardial contraction, i.e., a heart beart.
- Determine whether the event is a useable event or an invalid event.
- Determine a used interval if two consecutive useable events are present in the present interval.
- Increment the termination counter if the used interval is below a predetermined duration.
- Set the termination counter to zero if the used interval exceeds the predetermined duration.
- Detect a termination of the high ventricular rate, if the termination counter exceeds a predetermined threshold.

A preferred embodiment of an asystole detection method comprises the following steps:
- Detect an event corresponding to a myocardial contraction.
- Determine whether the event is a useable event or an invalid event.
- Determine a used interval if two consecutive useable events are present in the present interval.
- Detect an asystole if the used interval is longer than a predetermined asystole interval limit.

A preferred embodiment of a Brady rate limit detection method comprises the following steps:
- Detect an event corresponding to a myocardial contraction.
- Determine whether the event is a useable event or an invalid event.
- Determine a used interval if two consecutive useable events are present in the present interval.
- Add the used interval to a sum of intervals.
- Determine whether the sum of intervals exceeds the predetermined bradycardia duration.
- An optional step can be to remove the oldest interval, if the duration of the sum of used intervals without the oldest interval exceeds the predetermined bradycardia duration.
- If the sum of intervals exceeds the predetermined bradycardia duration, determine an average duration of the summed used intervals.
- Detect bradycardia, if the average interval is more than the predetermined duration.

One embodiment of the Brady rate limit detection method comprises the step of removing the oldest used interval from the sum of used intervals, if an event has a noise condition (NC) and/or a low signal indication, i.e., if an invalid event is detected.

A preferred embodiment of a Brady rate drop detection method comprises the following steps:
- Detect an event corresponding to a myocardial contraction.
- Determine whether the event is a useable event or an invalid event.
- Determine a used interval if two consecutive useable events are present in the present interval.
- Add the used interval to a sum of intervals.
- Determine whether the sum of intervals exceeds the sum of a pre-interval number of used intervals and a post-interval number of used intervals.
- If the sum of intervals exceeds the sum of the pre-interval number of used intervals and the post-interval number of used intervals, determine a pre-interval average and a post-interval average.
- Compare pre-interval average and post-interval average to determine a change in the average rate of events.
- Detect bradycardia, if the average rate of events decreases by a predetermined percentage threshold, i.e., if the post-interval average is larger than a predetermined fractional of the pre-interval average.

One embodiment of the Brady rate drop detection method comprises the step of removing the oldest used interval from the sum of used intervals, if an event has a noise condition (NC) and/or a low signal indication, i.e., if an invalid event is detected.

A preferred embodiment of a bradycardia termination detection method comprises the following steps:
- Initialize bradycardia termination detection method only if bradycardia was detected and set a bradycardia termination counter to zero.
- Detect an event corresponding to a myocardial contraction. Determine whether the event is a useable event or an invalid event.
- Determine a used interval if two consecutive useable events are present in the present interval. Increment the bradycardia termination counter if the used interval is shorter than the interval equivalent of the predetermined Brady rate limit.
- An optional step can be to decrement the bradycardia termination counter if an invalid event is detected and the bradycardia termination counter is greater than zero.
- Set the bradycardia termination counter to zero if the used interval is longer than or equal to the interval equivalent of the predetermined Brady rate limit.
- Detect termination of bradycardia if the predetermined bradycardia termination counter exceeds a predetermined threshold.

Another embodiment of the Brady rate drop detection method comprises the step of removing used intervals from the sum of used interval following a predetermined number of invalid events. The number of useable intervals can be set to 0 if an event has a noise condition (NC) and/or a low signal indication. The predetermined invalid event count may, for example, be 1, 2, 3 or 4. A default value for the predetermined invalid event count is preferably 1.

The foregoing and other objects, advantages and novel features of the present invention can be understood and appreciated by reference to the following detailed description of the invention, taken in conjunction with the accompanying drawings, in which:
- Fig. 1: shows a remote monitoring system and a heart stimulator;
- Fig. 2: illustrates a heart stimulator connected to electrode leads that are placed in a heart;
- Fig. 3: depicts a schematic block diagram of some components of the heart stimulator of figure 2;
- Fig. 4: shows input and output interfaces of a high ventricular rate (HVR);
- Fig. 5: shows a flow chart of a HVR algorithm for detection and termination of a HVR;
- Fig. 6: shows two intervals of an exemplary evaluation of a high ventricular rate (HVR);
- Fig. 7: shows a first exemplary evaluation of a high ventricular rate (HVR) with HVR detection;
- Fig. 8: shows a first exemplary evaluation of a high ventricular rate (HVR) without HVR detection;
- Fig. 9: shows a first exemplary evaluation of a high ventricular rate (HVR) with HVR termination;
- Fig. 10: shows a second exemplary evaluation of a high ventricular rate (HVR) with HVR termination;
- Fig. 11: shows an example of a detection of an asystole;
- Fig. 12: shows a first exemplary evaluation of a Brady rate limit without detection of bradycardia;
- Fig. 13: shows a second exemplary evaluation of a Brady rate limit with an interval sum below a programmed duration;
- Fig. 14: shows a third exemplary evaluation of a Brady rate limit with an interval sum below a programmed duration;
- Fig. 15: shows a fourth exemplary evaluation of a Brady rate limit with an interval sum below a programmed duration;
- Fig. 16: shows a fifth exemplary evaluation of a Brady rate limit with an average rate of events indicative of bradycardia;
- Fig. 17: shows a first exemplary evaluation of a Brady rate drop with detection of bradycardia;
- Fig. 18: shows a second exemplary evaluation of a Brady rate drop with a too small number of intervals;
- Fig. 19: shows a third exemplary evaluation of a Brady rate drop with a too small number of intervals; and
- Fig. 20: shows a fourth exemplary evaluation of a Brady rate drop without detection of bradycardia.

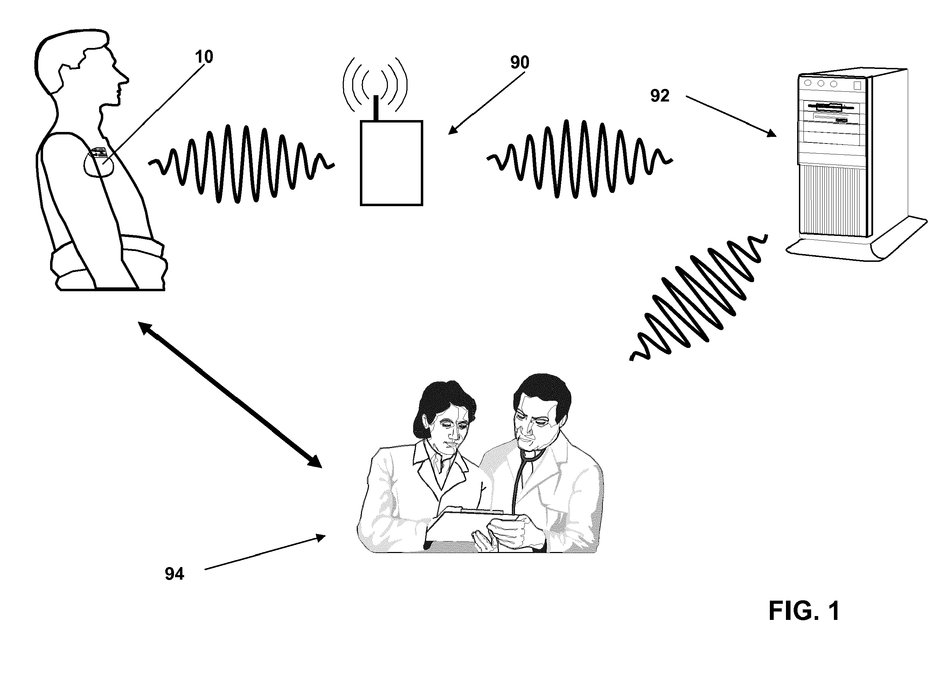

In Figure 1, a remote monitoring system including an implantable heart monitor or stimulator 10, an external device 90 and a central data server 92 of a central service center is displayed. Such system allows data communication between the implantable heart monitor and stimulator 10 and central server 92 via the external device 90. External device 90 is configured to communicate wirelessly with implantable heart monitor and stimulator 10.

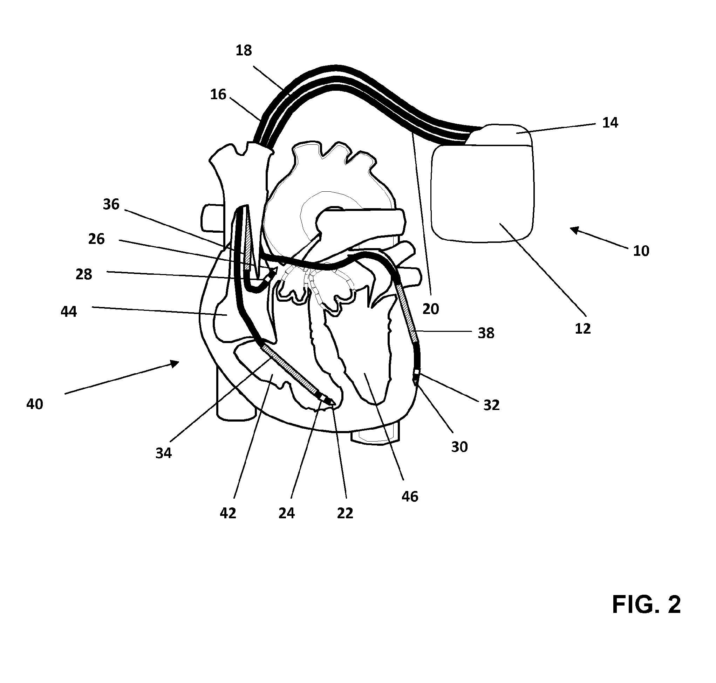

From figure 2 it is apparent that implantable heart monitor and stimulator 10 comprises a housing or case 12 and a header 14.

The implantable heart monitor and stimulator 10 is connected to three electrode leads, namely a right ventricular electrode lead 16, a right atrial electrode lead 18 and a left ventricular electrode lead 20.

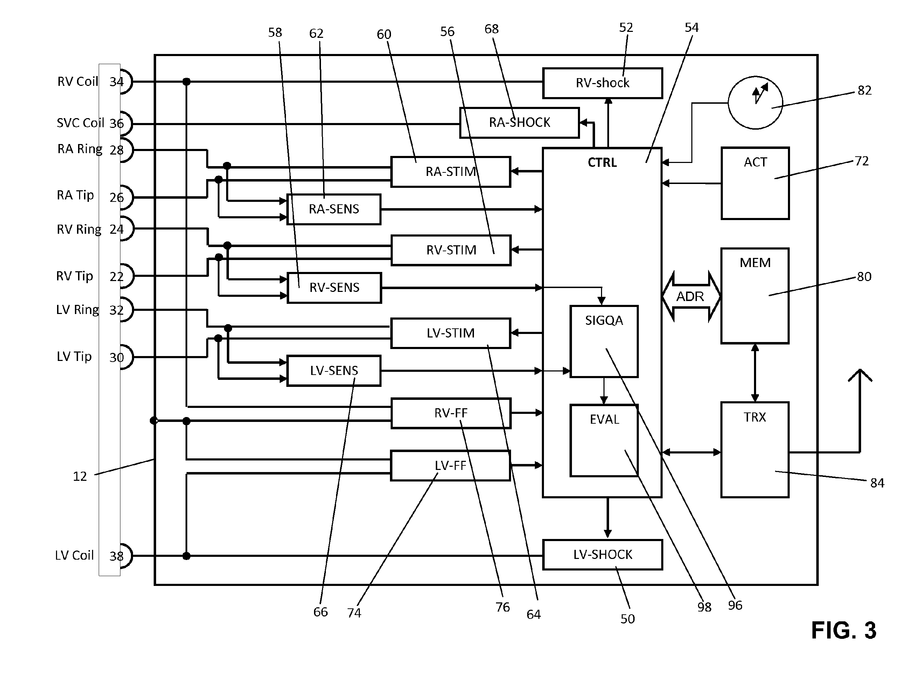

Figures 2 and 3 illustrate the pacing system that includes the implantable heart monitor and stimulator 10 and the connected leads 16, 18, and 20. The right atrial electrode lead 18 has a distal right atrial tip electrode 26 (RA-tip) at the distal end of right atrial electrode lead 18 and a proximal right atrial ring electrode 28 (RA-ring), as well as a superior vena cava coil electrode 36 (SVC-coil) that has large surface area.

The right ventricular electrode lead 16 has a distal right ventricular tip electrode 22 (RV-tip) at the distal end of right ventricular electrode lead 16 and a proximal right ventricular ring electrode 24 (RV-ring), as well as a right ventricular defibrillation coil electrode 34 (RV-coil) that has large surface area.

Similarly, the left ventricular (LV) lead has a distal left ventricular tip electrode 30 (LV-tip) and a proximal left ventricular ring electrode 32 (LV-ring), as well as a defibrillation coil electrode 38 (LV-coil) that has large surface area. The left ventricular electrode lead 20 is designed to pass trough the coronary sinus of heart 40.

Each electrode and shock coil of electrode leads 16 to 20 is separately connected to an electric circuit enclosed by case 12 of heart stimulator 10 by way of electrical contacts of a plug (not shown) at the proximal end of each electrode lead 16 to 20 and corresponding contacts (not shown) in header 14 of heart stimulator 10.

Now refer to Figure 3. SVC shock coil 36 is connected to right atrial shock generator 68 that is controlled by a control unit 54 of heart stimulator 10.

Similarly, right ventricular shock coil 34 is connected to a right ventricular shock generator 52 that is connected to control unit 54 and left ventricular shock coil 38 is connected to a left ventricular shock generator 50 that is also connected to control unit 54.

Right atrial tip electrode 26 and right atrial ring electrode 28 are both connected to a right atrial stimulation pulse generator 60 and a right atrial sensing stage 62 that are internally both connected to control unit 54.

Right atrial stimulation pulse generator 60 is adapted to generate atrial stimulation pulses of sufficient strength to cause an excitation of atrial myocardium by an electrical pulse delivered via right atrial tip electrode 26 and right atrial ring electrode 28. Preferably, means are provided to adapt the right atrial stimulation pulse strength to the stimulation threshold in the right atrium.

Right atrial sensing stage 62 is adapted to pick up myocardial potentials indicating an intrinsic atrial excitation that corresponds to a natural atrial contraction. By way of right atrial sensing stage 62, it is possible to stimulate the right atrium 44 of heart 40 in a demand mode wherein a right atrial stimulation pulse is inhibited if an intrinsic atrial event (intrinsic atrial excitation) is sensed by right atrial sensing stage 62 prior to expiration of an atrial escape interval.

In a similar manner, right ventricular ring electrode 24 and right ventricular tip electrode 22 are connected to right ventricular stimulation pulse generator 56 and to a right ventricular sensing stage 58 that in turn are connected to control unit 54. The right ventricular sensing stage 58 is further connected to a signal quality analysis unit 96 of the control unit 54, which determines whether a noise condition (NC) and/or a low signal indication is present for an intrinsic ventricular event sensed by the right ventricular sensing stage 58. The signal quality analysis unit 96 generates a noise condition and/or low signal indication signal and provides it to an evaluation unit 98. By way of right ventricular tip electrode 22, right ventricular ring electrode 24, right ventricular stimulation generator 56 and right ventricular sensing stage 58, right ventricular stimulation pulses can be delivered in a demand mode to the right ventricle 42 of heart 40.

In the same way left ventricular tip electrode 30 and left ventricular ring electrode 32 are connected to the left ventricular stimulation pulse generator 64 and the left ventricular sensing stage 66 that is connected to signal quality analysis unit 96 of the control unit 54 and that allow for stimulating a left ventricle 46 of heart 40.

The outputs of the ventricular sensing states 58 and 66, i.e., sense signals comprising ventricular events, and of the signal quality analysis unit 96, i.e., noise condition and/or low signal indication signals, are provided to the evaluation unit 98. The evaluation unit 98 evaluates the signals by detecting useable intervals in the sense signals in dependence of the noise condition and/or low signal indication signals. Useable intervals are intervals, which are defined by two consecutive events that do not have a noise condition and/or a low signal indication. The evaluation unit 98 executes various monitoring and stimulation algorithms in parallel to monitor specific heart functional and rhythm disorders, e.g., bradycardia, asystole, high ventricular rate, or other heart functional or rhythm disorders and to treat the disorder, e.g., by stimulating the left ventricle 46 and/or the right ventricle 42 of heart 40. The evaluation unit 98 determines an average interval duration and an average rate of events from the useable intervals and uses these parameters to detect bradycardia, asystole, and/or high ventricular rate. If a functional disorder has been detected the evaluation unit 98 executes an alternative monitoring and stimulation algorithm for the specific functional disorder which has been detected. The alternative monitoring and stimulation algorithm attempts to detect, whether the functional disorder was terminated. A termination may occur caused by stimulating the left ventricle 46 or right ventricle 42 of heart 40. The detection of termination and a stimulation adjusted to a detected functional disorder can also be integrated in one monitoring and stimulation algorithm.

Triggering and inhibition of delivery of stimulation pulses to the right atrium, the right ventricle or the left ventricle is controlled by control unit 54, in a manner known to the man skilled in the art. The timing that schedules delivery of stimulation pulses if needed is controlled by a number of intervals that at least partly may depend on a hemodynamic demand of a patient that is sensed by means of an activity sensor 72 that is connected to control unit 54. Activity sensor 72 allows for rate adaptive pacing wherein a pacing rate (the rate of consecutive ventricular stimulation pulses for a duration of consecutive atrial stimulation pulses) depends on a physiological demand of a patient that is sensed by a way of activity sensor 72.

A clock 82 allows recording of events and signals in association with time stamps that enable a synchronous evaluation of signals at a later point of time.

For the purpose of composition of a far-field right ventricular electrogram (RV EGM) and a far-field left-ventricular electrogram (LV EGM) a far-field right ventricular electrogram recording unit 76 and a far-field left ventricular recording unit 74, respectively, are provided. The far-field right ventricular electrogram recording unit 76 is connected to a case electrode that is formed by at least an electrically conducting part of case 12 of the heart stimulator 10 and to the RV coil electrode 34. The far-field left ventricular recording unit 74 is also connected to the case electrode formed by a case 12 of heart stimulator 10 and to the left ventricular coil electrode 38.

The near-field electrogram in the right ventricle 42 is measured between the RV-tip electrode 22 and RV-ring electrode 24. Preferably, the far-field electrogram in the right ventricle 42 is measured between the RV-coil electrode 34 and the device housing 12. Alternatively, the far-field electrogram in the right ventricle 42 can be measured between the RV-ring electrode 24 and the device housing 12.

Likewise, the near-field electrogram in the left ventricle 46 is measured between the LV-tip electrode 30 and LV-ring electrode 32. Preferably, the far-field electrogram in left ventricle is measured between the LV-coil electrode 38 and the device housing 12. Alternatively, the far-field electrogram in the left ventricle 46 can be measured between the LV-ring electrode 32 and the device housing 12.

Preferably, the far-field electrogram in the right ventricle 42 and the left ventricle 46 are minimally filtered and have wide bandwidth, e.g., with lower corner frequency 4 Hz and high corner frequency 128 Hz, whereas the near-field electrograms in the right ventricle 42 and the left ventricle 46 are filtered with narrower bandwidth, e.g., with lower corner frequency 18 Hz and high corner frequency 40 Hz. Accordingly, right and left far-field ventricular recording units 76 and 74 comprise each a band pass filter with lower corner frequency 4 Hz and high corner frequency 128 Hz. Right ventricular sensing stage 58 and left ventricular sensing stage 66 for picking up near-field electrograms in the right ventricle 42 and the left ventricle 46 each comprise band-pass filters with narrower bandwidth, e.g., with lower corner frequency 18 Hz and high corner frequency 40 Hz.

Both the far-field electrograms and the near-field electrograms can be used to detect events in the signals and to determine intervals and/or a corresponding a rate of events. The signal quality analysis unit 96 determines whether a noise condition (NC) and/or a low signal indication is present for an intrinsic event sensed by the sensing stages 58, 66 and/or the far-field ventricular electrogram recording units 74, 76. The corresponding noise condition (NC) and/or low signal indication signal is provided to the evaluation unit 98. The evaluation unit 98 evaluates the outputs of the sensing stages 58, 66 and the far-field ventricular electrogram recording units 74, 76 in dependence of the noise condition (NC) and/or low signal indication signal, i.e., determining an average interval duration and an average rate of events from the useable intervals and using these parameters to detect bradycardia, asystole, and/or high ventricular rate.

The heart monitor 10 is an implantable device, used as a loop recorder, that detects QRS complexes using the subcutaneous electrodes 22, 24, 30, 32 presented in Fig. 2 and Fig. 3. The heart monitor 10 combines different electrode measurements to create a combined signal and then performs QRS detection on this combined signal. The detected QRS events can be classified as ventricular sense events (VS) 102 or as invalid sensed events (VN) 104. A VS 102 is considered a VN 104 if it has an associated noise condition (NC) or low signal indication.

In the following a High Ventricular Rate (HVR) detection unit for use in the heart monitor 10 is described, which implements an algorithm with additional handling for invalid sensed events (VN) 104. The HVR noise handling mechanism is described in the following sections for detection and termination respectively.

### High Ventricular Rate unit

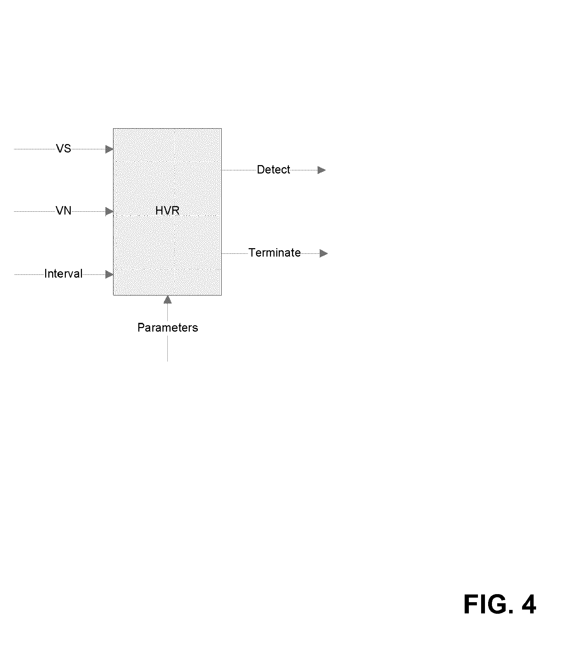

HVR uses event type, interval and parameters as inputs. See the following subsections for a description of these inputs. The output of HVR unit is detection or termination. The HVR input and output interfaces are shown in the figure 4.

Input and output parameters of the High Ventricular Rate unit are:
Event Types

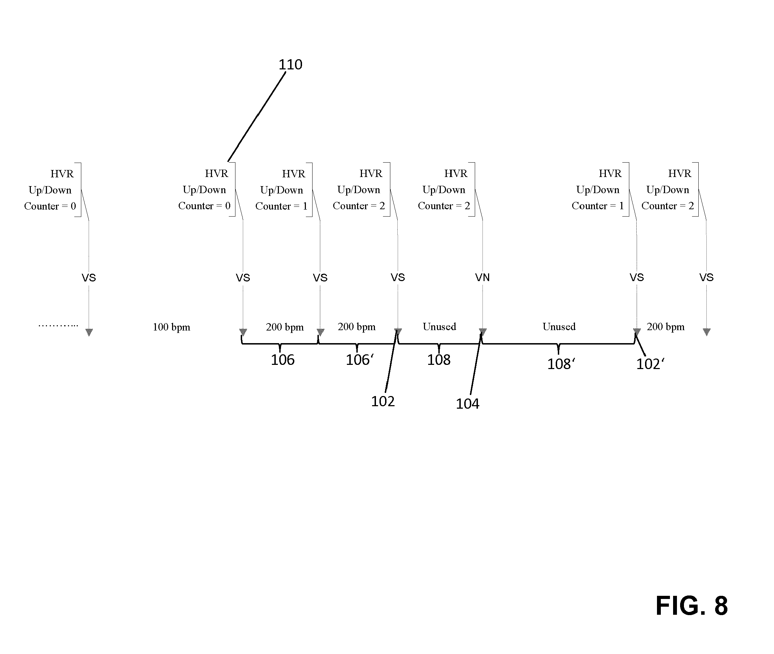

The heart monitor 10 has 2 event types, VS (used, respectively useable) 102 and VN (unused, respectively unuseable) 104, see figure 8. When an implant software (ISW) executed on the control unit 54 calls HVR, the implant software (ISW) provides classification of the event type as either VS 102 or VN 104.

### Intervals

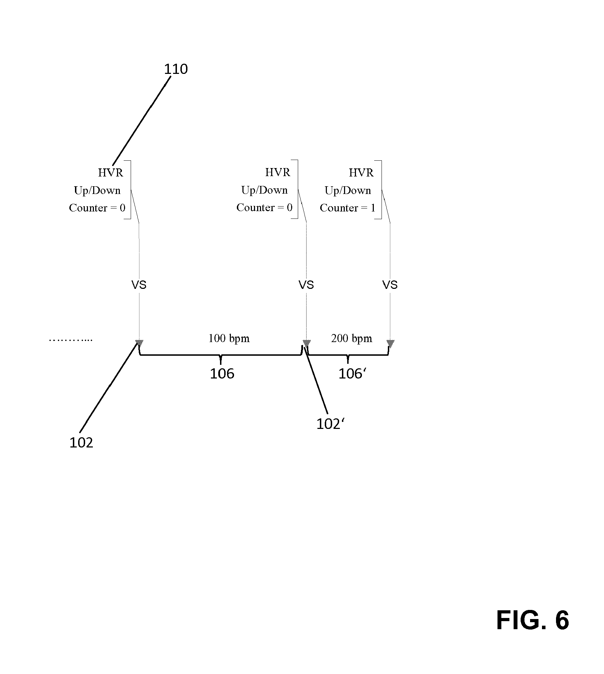

A used interval 106 is the interval duration measured between 2 consecutive VS 102, 102' events, see figure 6. An interval is considered unused 108, 108' if at least 1 of the 2 consecutive events making up the interval is a VN 104, see figure 8. It is assumed that when the implant software (ISW) calls HVR, the implant software (ISW) provides the most recent interval, if the interval is a used interval 106. The HVR unit will then test this interval to determine if it meets a high rate criterion. If the high rate criterion is met a high rate detection counter 110 will be incremented, see figure 6. If the implant software (ISW) provides an unused interval 108', the high rate detection counter 102 will be decremented if the first event of the interval is a VN 104, see figure 8. Unused intervals 108, 108' will not be tested for high rate conditions and will not affect termination.

### Parameters

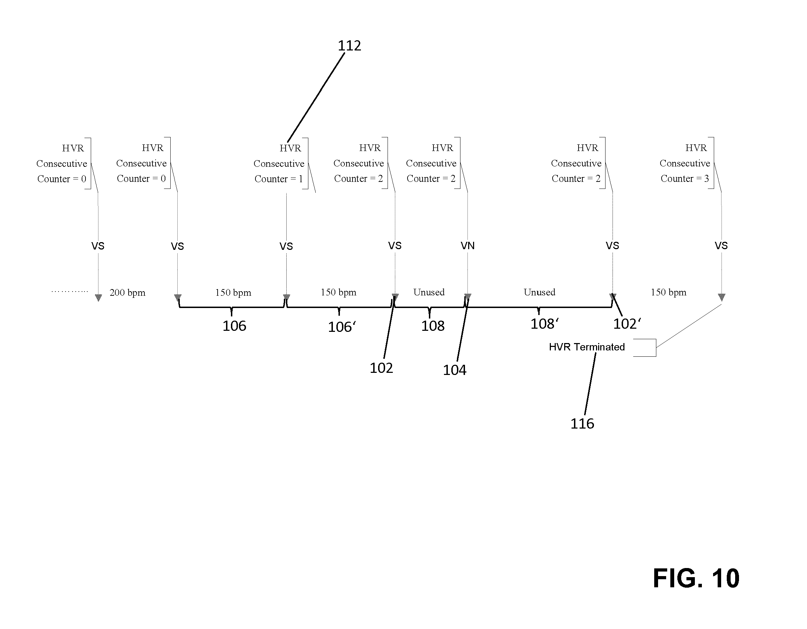

High ventricular rate (HVR) is detected when the high rate detection counter 110 exceeds a programmed threshold. See below for details of detection. The high rate detection counter 110 is incremented each time a rate above a programmed rate limit isdetected. The programmed rate limit is programmable in the range from 150 to 200 bpm in steps of 10 bpm. The default value is 180 bpm. Instead of incrementing the high rate detection counter 110 by exceeding the programmed rate limit, the high rate detection counter 110 can also be incremented when a used interval exceeds a detection interval limit. In the present case the detection interval limit corresponds to the programmed rate limit, i.e., the detection interval limit is programmable in the range from 0.3 s to 0.4s and has a default value of 0.33 s. The rate is the inverse to the interval. The threshold for the high rate detection counter 110 is programmable in the range from 4 to 16 in steps of 4. The default value is 8. Once HVR detects, HVR is declared and HVR changes mode to attempt to terminate HVR. HVR is terminated when the rate is below the programmed rate limit for a programmed number of consecutive used intervals 106. Unused intervals 108 play no role in termination and do not interrupt the calculation of consecutive used intervals, see figure 10. See section below for details of termination. The programmed number of consecutive used intervals 106 is programmable in the range from 4 to 16 in steps of 1. The default value is 5.

### High Ventricular Rate Algorithm

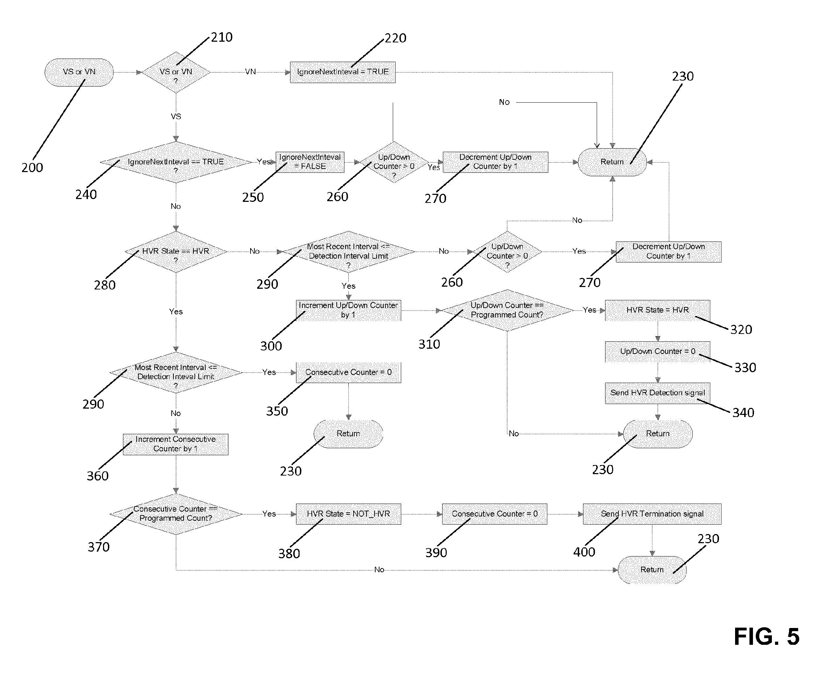

High ventricular rate (HVR) is detected when the high rate detection counter 110 exceeds the programmed threshold. The high rate detection counter 110 is incremented for used intervals 106 with a rate above the programmed rate threshold and decremented when unused intervals 108' are provided by the implant software (ISW). Once HVR detects, then HVR is declared and HVR changes mode to detect termination. HVR is terminated when the rate is below the programmed rate limit for a programmed number of consecutive used intervals 106. A detailed flow chart of the HVR algorithm is shown in the figure 5.

The HVR algorithm presented in figure 5 comprises the following steps:
- 200: Detect a QRS complex, i.e., a QRS event, in the combined electric signal.
- 210: Classify the QRS event as either ventricular sensed event VS 102 or invalid sensed event VN 104.
- 220: Set an ignore next interval flag to true, if QRS event is classified as VN 104.
- 230: Return to the start of the algorithm, i.e., step 200.
- 240: Check if the ignore next interval flag is set true.
- 250: Set the ignore next interval flag to false, if the ignore next interval flag was set as true.
- 260: Check if the high rate detection counter 110 is greater than 0.
- 270: Decrement the high rate detection counter 110 by 1, if the high rate detection counter 110 is greater than 0.
- 280: Check if a HVR state is detected, i.e., check if HVR state flag is true.
- 290: Check if the most recent interval is smaller than or equal to the detection interval limit.
- 300: Increment the high rate detection counter 110 by 1, if the most recent interval is smaller than or equal to the detection interval limit.
- 310: Check if the high rate detection counter 110 is equal to the programmed count.
- 320: Set the HVR state flag to true, if the high rate detection counter 110 is equal to the programmed count.
- 330: Set the high rate detection counter to 0, if the high rate detection counter 110 is equal to the programmed count.
- 340: Send a HVR detection signal, if the high rate detection counter 110 is equal to the programmed count.

The HVR detection signal may be used for other devices, e.g., stimulators to perform stimulation according to, e.g., a HVR stimulation scheme, or to a drug injection device to release drugs for treating the high ventricular rate.
- 350: Set a termination counter 114, i.e. consecutive counter, to 0, if the most recent interval is smaller than or equal to the detection interval limit.
- 360: Increment the termination counter 114 by 1, if the most recent interval is not smaller than or not equal to the detection interval limit.
- 370: Check if the termination counter 114 is equal to the programmed count.
- 380: Set a HVR state flag to false, i.e., set a HVR state to not HVR, if the termination counter 114 is equal to the programmed count.
- 390: Set the termination counter 114 to 0, if the termination counter 114 is equal to the programmed count.
- 400: Send a HVR termination signal, if the termination counter 114 is equal to the programmed count.

### Detection

The High ventricular rate (HVR) unit uses the high rate detection counter 110 for detection, which works as an up/down counter. Each used interval 106 has an equivalent rate greater than or equal to the programmed rate limit increases the high rate detection counter 110. Each used interval 106 that has an equivalent rate below the programmed rate limit decreases the high rate counter 110. Each unused interval 108 and 108' also decreases the high rate counter 110. This is to prevent HVR detection from occurring when only a few good intervals 106 are present during long periods of noise. The resulting used intervals 106 may have little relationship to each other in time in these circumstances. The result is a more specific HVR detection. When the high rate detection counter 110 reaches the programmed number of used intervals 106, then HVR is detected.

In figures 6 to 10 the most recent event is on the right and the oldest event is on the left. The HVR rate limit is representively programmed to 180 bpm, the number of used intervals 106 is representively programmed to 3 and the consecutive number of used intervals 106 is representively set to 3 for figures 6 to 10.

In figures 6 to 8, the high rate detection counter 110 used for detection is shown as HVR up/down counter.

### Used Interval Handling

In figure 6 a new used interval 106' is acquired, and then the HVR high rate detection counter 110 is incremented. The short interval 106' causes the HVR high rate detection counter 110 to increment with a rate limit programmed to 180 bpm.

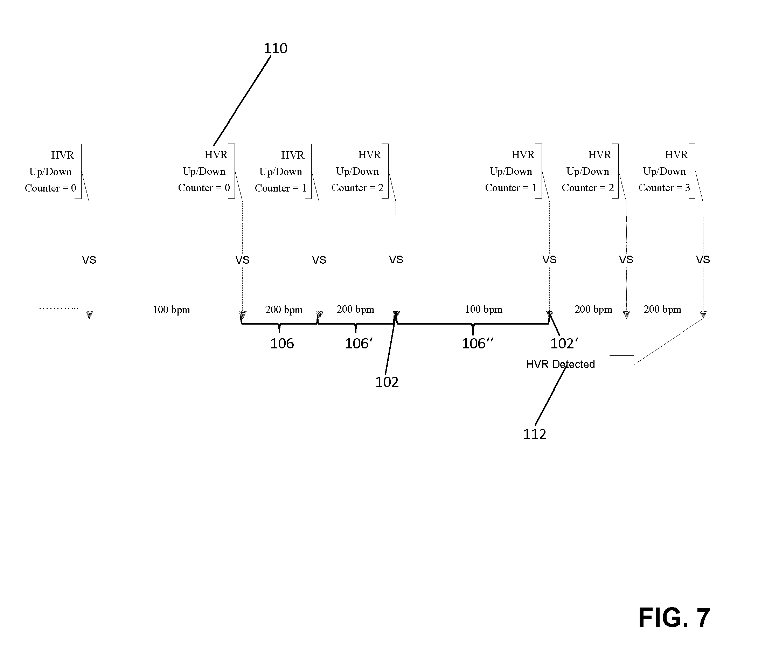

In figure 7 the high rate detection counter 110 is incremented twice due to 2 fast intervals 106, 106', then decremented once (due to 1 slower interval 106") and then incremented twice resulting in an HVR detection 112. HVR is detected with only used intervals 106 with a rate limit programmed to 180 bpm and the number of used intervals 106 programmed to 3.

### Unused Interval Handling

Intervals created using a VN 104 at the start of the interval 108' cause the high rate detection counter 110 to be decremented. See section above for details.

In figure 8 the high rate detection counter 110 is incremented twice due to 2 fast intervals 106, 106', then remains unchanged for an interval 108 created with a detected VN 104 and the high rate detection counter 110 is decremented once for the following interval 108' (due to 2 unused intervals 108, 108') and then incremented once resulting in no HVR detection. The HVR is not detected with intermittent unused intervals 108, 108' with a rate limit programmed to 180 bpm and the number of used intervals 106 programmed to 3.

In an alternative embodiment the intervals 108, 108' created using a VN 104 can cause the high rate detection counter 110 to remain unchanged. In this case the high rate detection counter 110 is incremented twice due to 2 fast intervals 106, 106', then remains unchanged for 2 intervals 108, 108' (due to 2 unused intervals) and is then incremented once resulting in HVR detection (not shown).

### Termination

Termination of HVR is attempted following detection of HVR. High ventricular rate (HVR) termination uses a termination counter 112, working as a consecutive counter. Each used interval 106 that has an equivalent rate less than the programmed rate limit increases the termination counter 114. Each used interval 106 that has an equivalent rate greater than or equal to the programmed rate limit clears the termination counter 114. When the termination counter 114 reaches the programmed number of consecutive used intervals 106, then HVR is terminated. Unused intervals 108 play no role in HVR termination.

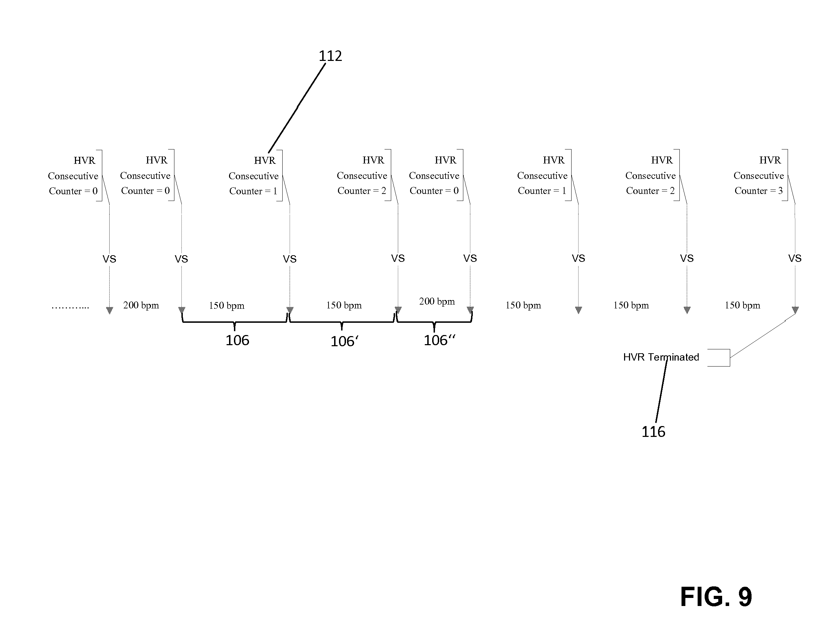

In figures 9 and 10 the termination counter 114, i.e., HVR consecutive counter, used for termination, is shown.

### Used Interval Handling

In figure 9 the termination counter 114 is incremented twice due to 2 slow intervals 106, 106', then reset to zero (due to 1 fast interval 106") and then incremented 3 times, resulting in an HVR termination 116. HVR is terminated with only used intervals 106 with a rate limit programmed to 180 bpm and the number of consecutive used intervals 106 programmed to 3.

### Unused Interval Handling

In figure 10 the termination counter 114 is incremented twice due to 2 slow intervals 106, 106', then remains unchanged for 2 intervals 108, 108' (due to 2 unused intervals 108, 108') and then is incremented once resulting in an HVR termination 116. HVR is terminated with intermittent unused intervals 108, 108' with a rate limit programmed to 180 bpm and the number of consecutive used intervals 106 programmed to 3.

### Brady and Asystole unit

This section describes the proposed Brady and Asystole detection unit for use in the heart monitor 10. Asystole is defined as a ventricular interval greater than or equal to 3 seconds, see, e.g., EHRA Position Paper "Indications for the use of diagnostic implantable and external ECG Loop recorders", Eurospace 11, 671-687 (2009). Bradycardia is defined as decrease of heart rate by 30%, Brady Rate Drop, or a heart rate lower than 40 bpm that is sustained longer than 10 seconds, Brady Rate Limit, see, e.g., EHRA Position Paper "Indications for the use of diagnostic implantable and external ECG Loop recorders", Eurospace 11, 671-687 (2009). Brady rate drop, Brady rate limit and asystole have a noise handling mechanism described in their sections respectively.

### Used Intervals

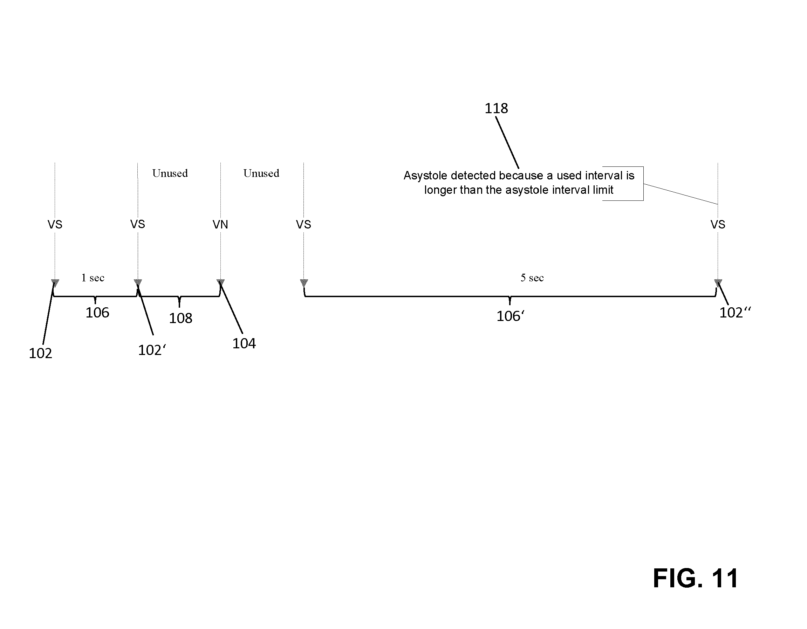

The heart monitor 10 works with the 2 event types, VS (used) 102 and VN (unused) 104. A used interval 106 is the interval measured between 2 consecutive VS events 102, 102', see figure 11. An interval 108 is considered unused if at least 1 of the 2 consecutive events making up the interval is a VN 104, see figure 11.

### Asystole

Asystole 118 is detected if a single usable interval 106' is longer than the programmed asystole interval limit, otherwise it is not detected, see figure 11. The asystole interval limit can be programmed in the range from 2 to 10 seconds in steps of 1 second. The default value is 3 seconds.

In figure 11 the most recent event is on the right and oldest event is on the left and the programmed asystole interval limit is 3 seconds.

In figure 11 the asystole 118 is detected on the VS 102" concluding the most recent interval 106' because the used interval 106' is longer than the programmed asystole interval limit.

### Bradycardia

Bradycardia can be detected by either Brady rate limit or Brady rate drop and run independently and in parallel until either one of them detects bradycardia. Once either algorithm detects, then bradycardia is declared and the bradycardia detection algorithm changes into a termination mode and attempts to detect termination.

### Brady Rate Limit

Brady rate limit is detected when the average rate is less than a programmed rate limit for a programmed duration. The rate limit can be programmed in the range from 30 to 80 bpm in steps of 5 bpm. The programmed duration can be programmed from 5 to 30 seconds in steps of 5 seconds. The default values are 40 bpm for 10 seconds.

### Detection

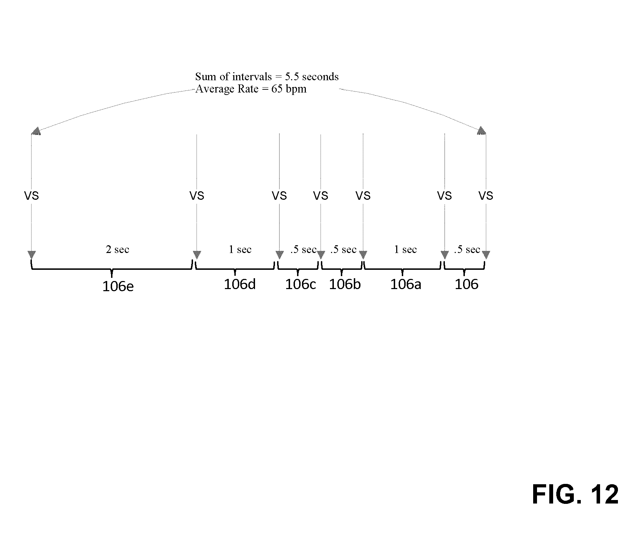

Used intervals 106, 106a, 106b, 106c, 106d, 106e are summed starting with the newest used interval 106, going back in time, to find the minimum number of consecutive used intervals 106 whose sum is greater than or equal to the programmed duration, see figure 12. The newest used interval 106 is always included in the previously mentioned sum. This sum is updated on every used interval 106. If there are enough used intervals, such that their sum is greater than or equal to the programmed duration, then the average of these intervals is computed and then converted to a rate. If this rate is less than the programmed rate limit, then Brady rate limit is detected, otherwise Brady rate limit is not detected. If the sum of all of the used intervals is less than programmed duration, then Brady rate limit is not detected. When Brady rate limit is initialized the number of usable intervals is set to zero.

In the figures 12 to 16 the most recent event is on the right and events get older from right to left and the programmed asystole duration is 5 seconds.

In figure 12 the sum of the used intervals is greater than the programmed duration, so the average rate is computed and compared to the programmed rate limit. The average rate is 65 bpm and therefore greater than the Brady rate limit, leading to no bradycardia detection.

### Unused Interval Handling

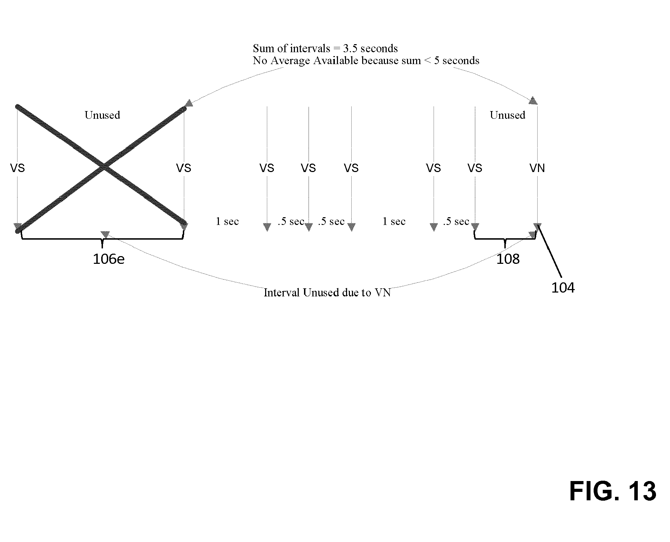

Following a VN 104, the Brady rate limit detection algorithm removes the oldest used interval 106e from the previously used intervals to calculate a new used-interval-sum. In figure 13 a VN 104 occurs which leads to removal of the oldest used interval 106e. In addition, the newest interval 108 is not added to the used-interval-sum because it is unused. Therefore the VN 104 causes the removal of 1 used interval.

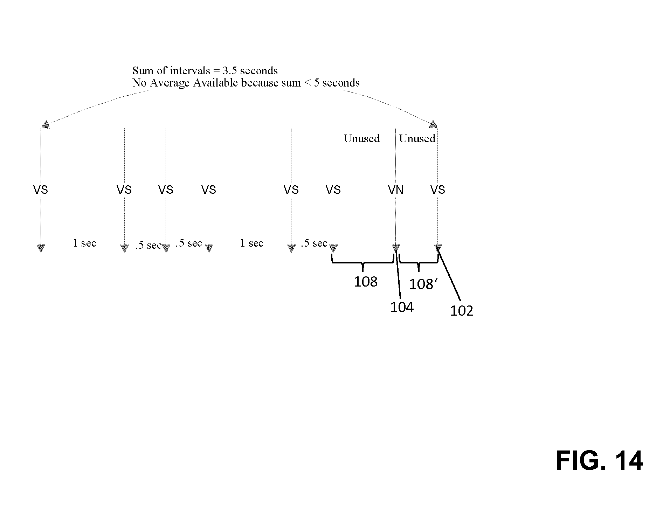

A VS 102 following a VN 104 does not produce a usable interval, but an unused interval 108', therefore the used-interval-sum is unchanged, as shown in figure 14. The usable intervals are the same in figure 13 and figure 14.

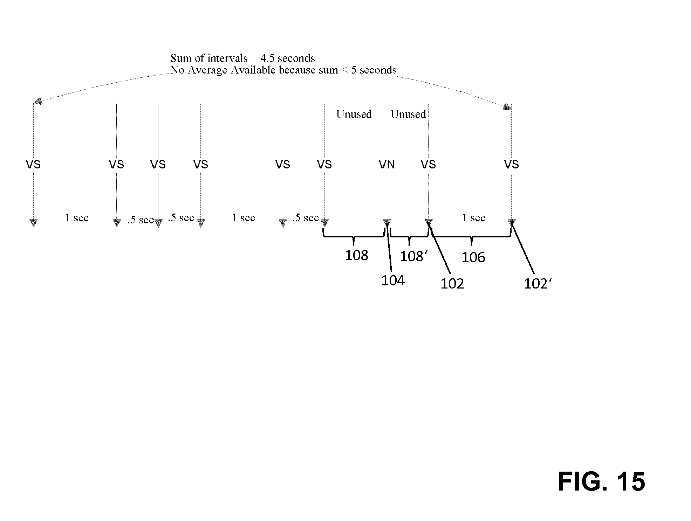

In figure 15 a new used interval 106 is acquired, then the used intervals are summed and the unused intervals 108, 108' are ignored in the used-interval-sum. The used-interval-sum is less than the programmed duration, so the average rate is not compared to the programmed rate limit.

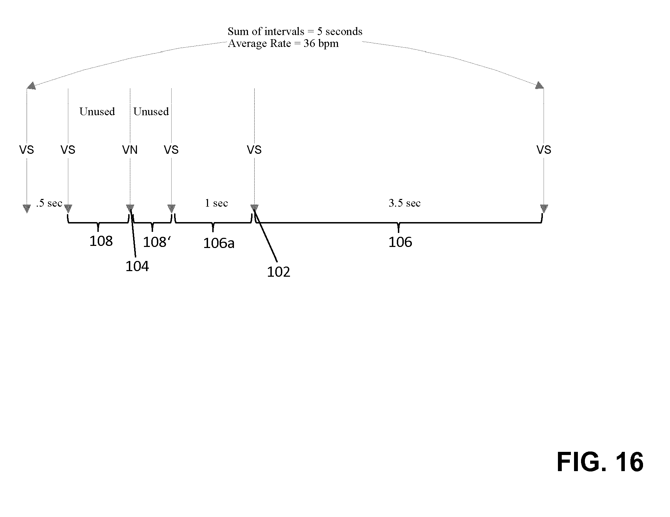

In figure 16 a new used interval 106 is acquired, then the used intervals are summed and the unused intervals 108, 108' are ignored in the used-interval-sum. The used-interval-sum is equal to the programmed duration, so the average rate is computed and compared to the programmed rate limit. The average rate is 36 bpm, which is below the default rate limit of 40 bpm for the Brady rate limit resulting in a detection of bradycardia.

### Brady Rate Drop

Brady rate drop is detected when the rate decreases by a programmed percentage. The change in rate is measured by comparing a pre-interval average 120 and a post-interval average 122. The number of intervals used in the pre-interval average 120 can be programmed to 32, 48 or 64. The number of intervals used in the post-interval average 122 can be programmed to 4, 8 or 16. The default values are 48, 8 and 30% for the pre-interval number, post-interval number and rate decrease percentage respectively.

### Detection

Brady rate drop can not be detected unless the total number of used intervals 106 is equal to the programmed pre-interval number plus the programmed post-interval number. The number of used intervals is set to zero upon initialization. For each new used interval 106, the number of used intervals is incremented as long as it does not exceed the pre-interval number plus post-interval number of used intervals, else the interval number equals the pre-interval number plus post-interval number.

The post-interval average 122 is calculated by taking the average of the most recent post-interval number of used intervals. The post-interval average 122 is always computed using the most recent used interval 106. The pre-interval average 120 is computed using the previous consecutive pre-interval number of used intervals preceding the intervals used to calculate the post-interval average. If there are pre-interval number plus post-interval number of used intervals, these averages are updated on every used interval 106. Both of these averages 120, 122 are then converted to a rate. If the post-interval average rate < (1 - programmed rate decrease percentage) * pre-interval average rate, then Brady rate drop is detected, else it is not.

Once Brady rate drop is detected the number of usable intervals is set to the programmed post-interval number. This effectively removes all intervals used in the pre-interval-average 120 from further Brady rate drop detections.

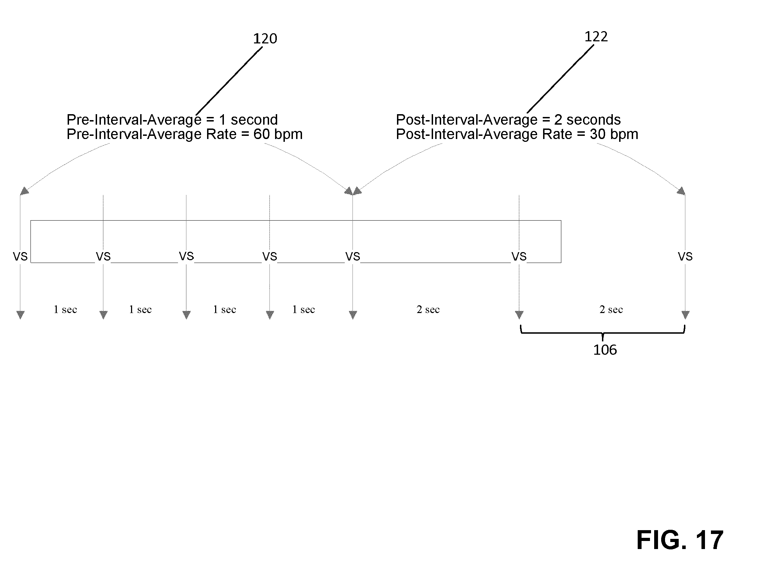

In the figures 17 to 20 the most recent event is on the right and events get older from right to left. The pre-interval number and the post-interval number are set to 4 and 2 respectively for figures 17 to 20. Values of 4 and 2 are not allowable settings in the implantable heart monitoring device 10, but they were chosen for illustrative purposes.

In figure 17 a new interval is acquired thereby filling the pre-interval buffer, and then the pre-interval averages 120 and post-interval averages 122 are computed. The rates derived from the pre-interval averages 120 and post-interval averages 122 lead to a pre-interval-average rate of 60 bpm and a post-interval-average rate of 30 bpm, a 50 % rate decrease, which would result in a detection of bradycardia for the default rate decrease percentage of 30%.

### Unused Interval Handling

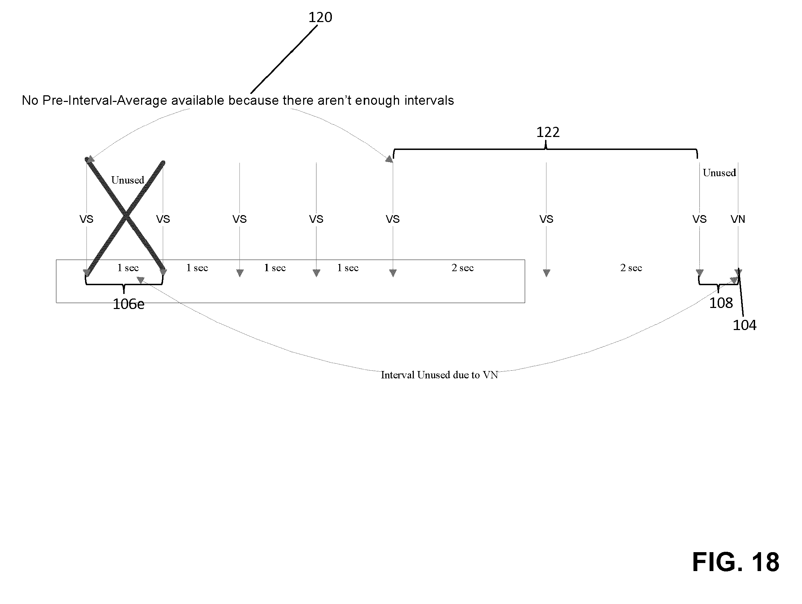

Following a VN 104, the Brady rate drop algorithm removes the oldest used interval 106e from the previously used intervals and decrements the usable interval count by 1. In figure 18 a VN 104 occurs which leads to removal of the oldest used interval 106e. In addition, the newest interval 108 is not added to the usable intervals because it is unused. Following the VN 104, the number of intervals is less than the programmed pre-interval number and the post-interval number. No pre-internal-average is available because there are not enough intervals.

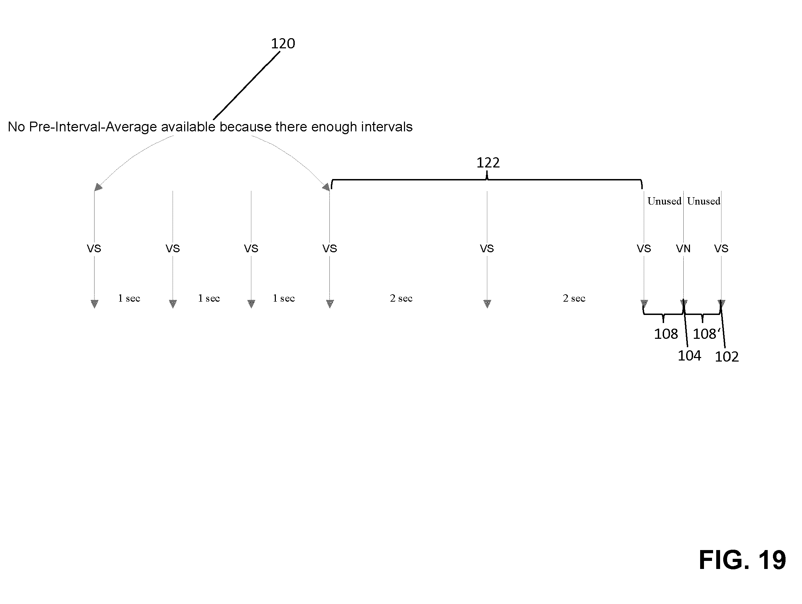

Figure 19 shows a VS 102 following a VN 104 does not produce a usable interval, but an unused interval 108', therefore the usable interval count is unchanged. The usable intervals are the same in figure 18 and figure 19. No pre-internal-average is available because there are not enough intervals.

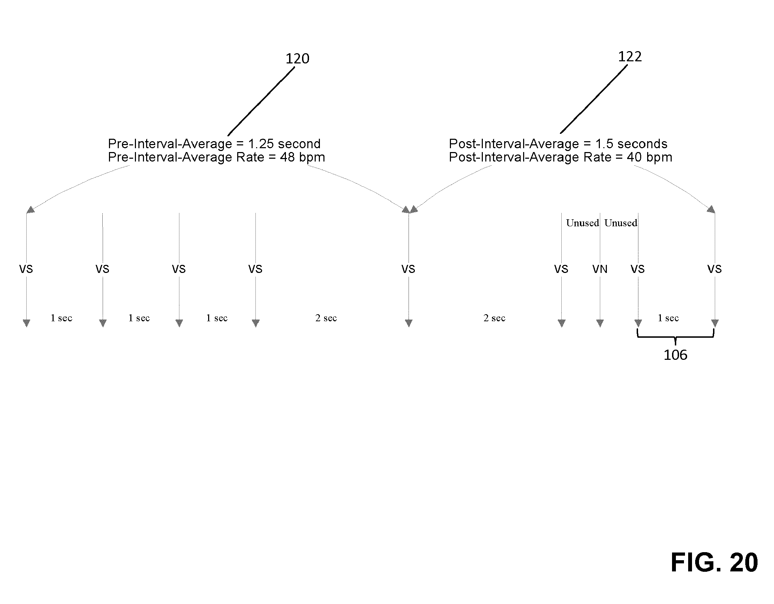

In figure 20 a new used interval 106 is acquired. Then, the pre-interval averages 120 and post-interval averages 122 are computed. The usable interval count equals the programmed pre-interval number plus the post-interval number, so the average rates are calculated and compared to see if Brady Rate Drop is detected. In the case presented in figure 20 no Brady rate drop is detected, as the rate decrease percentage is below 30%.

### Termination

Once bradycardia is detected by either Brady rate limit or Brady rate drop, the algorithm changes into the termination mode and attempts to detect termination. Bradycardia is terminated after a bradycardia termination counter reaches 10. The bradycardia termination counter is initialized to zero on detection of Brady rate limit or Brady rate drop. The termination count is incremented for each used interval shorter than the interval equivalent of the programmed rate limit. The programmed rate limit used for Brady rate limit detection is the same rate limit used for termination of Brady rate limit and Brady rate drop. The termination count is reset to zero for each used interval that is longer than or equal to the interval equivalent of the programmed rate limit. The termination counter is decremented for each VN 104, if the termination count is greater than zero; otherwise the termination counter remains zero.

## Claims

1. An implantable heart monitor (10) comprising
- a sensing unit (58, 62, 66) which can be connected or is connected to at least one electrode (22, 24, 24, 26, 30, 32) for picking up electric potentials, wherein the sensing unit (58, 62, 66) is adapted to process electrical signals corresponding to said electric potentials, to detect signal components representing a myocardial contraction, and to generate sense signals representing detected events (102, 104) corresponding to myocardial contractions,
- a signal quality analysis unit (96) configured to determine whether a noise condition (NC) and/or a low signal indication is present for an event (102, 104) and to generate a noise condition and/or low signal indication signal, and
- an evaluation unit (98) connected or connectable to said sensing unit (58, 62, 66) and said signal quality analysis unit (96) and configured to evaluate sense signals and noise condition and/or low signal indication signals,
wherein said evaluation unit (98) is configured to treat the detected events (104) as non-valid if a noise condition (NC) and/or low signal indication signal is present for that event (104) and to use only intervals (106) defined by two consecutive events (102) which do not have a noise condition (NC) and/or a low signal indication to determine a rate of events of the sense signal.

2. An implantable heart monitoring device (10) according to claim 1, wherein the sensing unit (58, 66) is a ventricular sensing unit (58, 66) configured to detect ventricular events (102, 104).

3. An implantable heart monitoring device (10) according to claim 2, wherein the evaluation unit (98) is configured to detect a high ventricular rate, if a high rate detection counter (110) exceeds a predetermined threshold, and wherein the evaluation unit (98) is configured to increment the high rate detection counter (110) each time the determined rate of events in a used interval (106) exceeds a predetermined threshold of a high ventricular rate detection limit.

4. An implantable heart monitoring device (10) according to claim 3, wherein the evaluation unit (98) is configured to decrement the high rate detection counter (110) each time the determined rate of events in a used interval (106) is below the predetermined threshold of the high ventricular rate detection limit.

5. An implantable heart monitoring device (10) according to claim 3 or 4, wherein the evaluation unit (98) is configured to decrement the high rate detection counter (110) each time an event (104) has a noise condition (NC) and/or a low signal indication.

6. An implantable heart monitoring device (10) according to at least one of the claims 3 to 5, wherein the evaluation unit (98) is configured to detect a termination (116) of the high ventricular rate, if the high ventricular rate was detected and a termination counter (114) exceeds a predetermined threshold, and wherein the evaluation unit (98) is configured to increment the termination counter (114) each time the determined rate of events in a used interval (106) is below a predetermined threshold of a high ventricular rate detection limit.

7. An implantable heart monitoring device (10) according to claim 6, wherein the evaluation unit (98) is configured to set the termination counter (114) to zero each time the determined rate of events in a used interval (106) exceeds the predetermined threshold of a high ventricular rate detection limit.

8. An implantable heart monitoring device (10) according to at least one of the claims 1 to 7, wherein the evaluation unit (98) is configured to detect an asystole (118) if a used interval (106) is longer than a predetermined asystole interval limit.

9. An implantable heart monitoring device (10) according to at least one of the claims 1 to 8, wherein the evaluation unit (98) is configured to detect bradycardia, if an average rate of events is less than a predetermined Brady rate limit for a predetermined bradycardia duration.

10. An implantable heart monitoring device (10) according to claim 9, wherein the evaluation unit (98) is configured to find a minimum number of used intervals (106) whose sum exceeds the predetermined bradycardia duration, to determine an average duration of the summed used intervals (106), to convert the average duration into an average rate of events, and to determine if the average rate of events is below the predetermined Brady rate limit.

11. An implantable heart monitoring device (10) according to claim 10, wherein the evaluation unit (98) is configured to add the newest used interval (106) to the minimum number of used intervals (106) when in use and if the sum of the used intervals (106) without the oldest interval (106e) exceeds the predetermined bradycardia duration, to remove the oldest used interval (106e) from the minimum number of used intervals (106).

12. An implantable heart monitoring device (10) according to at least one of the claims 10 or 11, wherein the evaluation unit (98) is configured to remove the oldest used interval (106e) from the minimum number of used intervals (106), if an event (104) has a noise condition (NC) and/or a low signal indication.

13. An implantable heart monitoring device (10) according to at least one of the claims 1 to 12, wherein the evaluation unit (98) is configured to detect bradycardia, if an average rate of events decreases by a predetermined percentage threshold, and wherein a change in the average rate of events is determined by comparing a pre-interval average comprising a predetermined pre-interval number of used intervals and a post-interval average comprising a predetermined post-interval number of used intervals.

14. An implantable heart monitoring device (10) according to at least one of the claims 8 to 13, wherein the evaluation unit (98) is configured to detect a termination of bradycardia, if bradycardia was detected and a predetermined bradycardia termination counter exceeds a predetermined threshold, wherein the bradycardia termination counter is incremented for each used interval shorter than the interval equivalent of the predetermined Brady rate limit, and wherein, if the bradycardia termination counter is greater than zero, the bradycardia termination counter is decremented for each event (104) which has a noise condition (NC) and/or a low signal indication.

15. An implantable heart monitoring device (10) according to claim 14, wherein the evaluation unit (98) is configured to set the bradycardia termination counter to zero for each used interval that is longer than or equal to the interval equivalent of the predetermined Brady rate limit.
